# EUROPEAN PATENT APPLICATION

(11) **EP 2 147 981 A1**
(43) Date of publication of application: **27.01.2010**
(21) Application number: 08161188.1
(22) Date of filing: 25.07.2008
(51) Int. Cl.: C12Q 1/68

(54) **Kit and method for evaluating detection properties in amplification reactions**

(71) Applicant: Biotype AG, 01109 Dresden (DE)
(72) Inventor: Brabetz, Werner, 01127 Dresden (DE); Gabert, Jörg, 04155 Leipzig (DE)
(74) Representative: Kilger, Christian

(57) **Abstract**

The invention relates to a kit comprising, (a) a first group of containers, comprising two or more containers with human genomic DNA, wherein the human genomic DNA in each of the two or more containers is of different human origin and the amount of DNA in the container is defined, (b) a second group of containers, comprising two or more containers with human genomic DNA, wherein the concentration of the DNA varies between the containers and the concentration of the DNA in the container is defined and (c) a third group of containers, comprising two or more containers with human genomic DNA, wherein the human genomic DNA in each container is a mixture of genomic DNA and is from at least two different individuals. The invention also relates to an evaluation tool and methods of use of the kit.

## Description

### FIELD OF THE INVENTION

The present invention is generally directed to a kit and methods for evaluating the detection limits of amplification agents more in particular polymerase chain reaction amplification agents. The invention is thus in the field of chemistry and biology, more particular in molecular biology, more particularly human genetics and most particularly in forensics as well as paternity testing.

### BACKGROUND OF THE INVENTION

DNA typing is commonly used to identify the parentage of human children and to confirm the linage of horses, dogs and other animals, and agricultural crops. DNA typing is also commonly employed to identify the source of blood, saliva, semen, and other tissue found at a crime scene or other sites requiring identification of human remains. DNA typing is also employed in clinical settings to determine success or failure of bone marrow transplantation in presence of particular cancerous tissues. DNA typing involves the analysis of alleles of genomic DNA with characteristics of interest, commonly referred to as "markers". Most typing methods in use today are specifically designed to detect and analyse differences in the length and/or sequence of one or more regions of DNA markers known to appear in at least two different forms in the population. Such length and/or sequence variations are referred to as "polymorphism". Any region, *i*.*e*. "locus" of DNA in which such variation occurs is referred to as "polymorphic locus".

When speaking about polymorphic DNA sequences one must distinguish in particular between the so called repeated polymorphic DNA sequences and non-repetitive polymorphic elements. In the study of DNA sequences one can distinguish two main types of repeated sequence tandem repeats and interspersed repeats. Tandem repeats include satellite DNA. Satellite DNA consist of highly repetitive DNA and is so called because repetitions of short DNA sequence tend to produce different frequency of the nucleotides adenine, cytosine, guanine and thymine and thus, have different density from bulk DNA - such that they form a second or a "satellite" band in genomic DNA separated on a density gradient. A mini-satellite is a section of DNA that consists of short series of bases 10 to 100 bp. These occur in more than 1,000 locations in the human genome.

Indispersed repetitive DNA is found in all eukaryotic genomes. These sequences propagate themselves by RNA mediated transposition and they have been called retroposons. Such retroposons are substantially larger than the repetitive elements discussed above.

So-called short indispersed nuclear elements (SINEs) are a further class of repetitive DNA elements. A particular type of SINEs are the so-called ALU-sequences. These are about 300 base pairs in length.

Micro satellites are simple sequence repeats (SSRs) or also short tandem repeats (STRs) or polymorphic loci present in nuclear DNA and organelle DNA that consist of repeating units of 1 to 6 base pairs in length. They are used as molecular markers which have wide-ranging applications in the field of genetics including kinship and population studies. Micro satellites can also be used to study gene dosage (looking for duplications or deletions of a particular genetic region). One common example of micro satellite is (CA)ₙ repeat, where n is variable between alleles. These markers are often present in high levels of inter- and intraspecific polymorphism. Particularly when tandem repeats number 10 or greater appear. The repeated sequences often simple, consisting of two, three or four nucleotides (di-, tri-, tetranucleotide repeats respectively) and can be repeated 10 to 100 times. CA nucleotide repeats are very frequent in human and other genomes, and are present every few thousand base pairs. As there are often extremely many alleles present at an STR locus, genotypes within pedigrees are often fully informative and the progenitor of a particular allele can often been identified.
When using, e.g. RFLP, the theoretical risk of a coincidental match is 1 in 100 billion (100,000,000,000). However, the rate of laboratory error is almost certainly higher than this, and often actual laboratory procedures do not reflect the theory under which the coincidence probabilities were computed. For example, the coincidence probabilities may be calculated based on the probabilities that markers in two samples have bands in precisely the same location, but a laboratory worker may conclude that similar-but not precisely identicaf-band patterns result from identical genetic samples with some imperfection in the agarose gel.

Systems containing several loci are called multiplex systems and many such systems containing up to more than 11 separate STR loci have been developed and are commercially available. Although, amplification protocols with STR loci can be designed which produce small products generally from 60 to 500 base pairs (bp) in length and alleles from each locus are often contained within range of less than 100 base pairs. Although, the analysis of multi-allelic short tandem repeats (STRs) still has the largest impact on forensic genetics and case work, it must be said, that the systems are limited especially for DNA evidences of low quality and quantity. For example degraded DNA samples represent one of the major challenges of the major STR analysis as amplicon sizes within multiplex assays often exceed 200 base pairs. Degraded samples are extremely difficult to amplify.

The polymorphisms mentioned above are used to associate or eliminate individuals as being the source of body fluid evidence often found in investigations of violent crimes. DNA has the ability to identify a person from a drop of blood the size of a pin head. DNA can be found practically anywhere on or in the human body. It is contained in blood, semen, saliva, skin cells, tissue, organs, muscle, bone, teeth, hair, urine, fingernails, sweat, faeces, and mucus. Physical evidence received in criminal cases by the laboratories is first analyzed for the presence of biological material such as blood or semen. Once a biological material is identified, the material is then analyzed in DNA. The DNA results are compared to the DNA profile of the victim and any potential suspects. If the suspect is eliminated or there is no suspect, the DNA results from the biological material are, in the USA entered into CODIS. CODIS is the Combined DNA Index System, overseen by the FBI.

Forensic investigators take many precautions to prevent mistakes, but human error can never be reduced to zero. The National Research Council (NRC) recommends that evidence samples be divided into several quantities soon after collection, so that if a mix-up were to occur, there would be backup samples to analyze. To detect possible contamination of DNA samples during collection or handling, evidence DNA profiles are often compared with those from detectives at the crime scene, the victim, a randomly chosen person or a DNA profile from a database. The NRC recommends that forensic DNA analysis be conducted by an unbiased outside laboratory that maintains a high level of quality control and a low error rate.

Scientific progress in this area has advanced rapidly over recent years and has resulted in different forensic laboratories using different techniques and typing systems. In addition, there are many different genetic markers that can be analysed. As a result, it has not only proven difficult to compare data from one laboratory with that of another, it is also difficult to check the quality of laboratory work. Quality may differ dramatically between laboratories.

In the European Union the clear need for standardisation was addressed in the 90-ies in a three-year programme of work being funded by the European Commission.

However, the following problems remain.

Avoidance and identification of contamination is a problem. As well as demonstrating provenance of the item or sample, one must be able to show that the DNA profile obtained actually came from DNA originally present in the item or sample and from nowhere else. It is important to recognise in this context that current DNA profiling methods are very sensitive. This increased sensitivity means that there is an increased risk that the DNA being analysed may not have come from the victim or offender, but from some other person not involved in the offence, either 'legitimately' from transfer before the offence or by contamination after the offence. Certain control samples would be able to identify such cases.

Ideally, everyone involved in the collection of items or samples from a scene of crime, or from suspects and victims, would wear appropriate barrier clothing at all times to prevent their contaminating the evidence. This would include gloves, masks, mob caps and scene of crime suits. All investigators attending a scene or dealing with suspects or victims would take similar precautions. This is not realistic and thus a test sample must be able to detect contamination.

Care must also be taken with the equipment and materials used to obtain samples. Traditional cleaning methods and even autoclaving do not always get rid of DNA. Specially designed sampling kits, with disposable 'DNA free' equipment and packaging, should therefore be employed at the crime scene, and by medical examiners and pathologists. Contamination may still happen. Specially designed test kits could be able to solve this problem.

For investigations into the more serious crimes it is likely that many of these precautions will be taken. However, it is accepted that avoidance of contamination may not be the prime consideration in other circumstances. In sexual assault cases, for example, sensitivity to the feelings of victims may be paramount. And in less serious investigations, such as those into auto crime or minor burglaries, fewer precautions may be used, simply on grounds of cost.

In the analysis process, contamination can occur from the equipment, or from the materials and reagents used, or from one sample to another, and it is essential to monitor for this.

Ensuring that the DNA profile obtained is correct is a problem. Best practice is for the laboratories to work to internationally accepted quality standards and to have in place for all their relevant activities a documented quality system containing all the protocols, methods and procedures that comply with these.

Bodies such as the ENFSI (European Network of Forensic Science Institutes) DNA Working Group and SWGDAM (Scientific Working Group on DNA Analysis Methods) have also produced guidelines for quality assurance, which cover in more detail all the management and technical requirements for the quality system. Most DNA laboratories work to these and in the United States the requirements are now enshrined in legislation.

The quality standards address the issues such as training, qualifications, competency assessment, evidence control, accommodation and environmental conditions that have been mentioned earlier. They also set out the requirements for calibration and testing, the minimum standards for carrying out DNA profiling, the interpretation and reporting of results, and audit and proficiency testing.

Validation and introduction of new equipment and techniques is a problem. It is good science and a requirement of the courts that all techniques on which evidence is based should be validated. For DNA techniques, this means that they have to be shown to be robust and to produce the correct results reliably and consistently. There has to be an understanding of their species specificity, their somatic stability and their sensitivity and behaviour when subjected to the sorts of environmental challenge and variations in analysis conditions encountered in casework. It is important to know how to deal with mixtures and one needs all the information necessary to be able to interpret results in a sound manner, for example the individual allele frequencies and the extent to which these are independent of one another. When validating and implementing new DNA techniques one should simultaneously introduce processes for on-going monitoring of the quality of the analysis. It is necessary to specify, for example, the requirements for equipment to be calibrated and monitored for performance within specification; for key reagents, other consumables and computer software to be tested before use, whether prepared in-house or purchased commercially.

Definitions, guidelines and directives for the quality management of test systems in testing and calibration laboratories are defined by the international standard *DIN EN ISO*/*IEC 17025:2005* (General requirements for the competence of testing and calibration laboratories). Accreditation in compliance with this standard is highly recommended or even claimed by national authorities or scientific associations for clinical, legal medicine, veterinary and forensic laboratories. According to this quality assurance systems validation experiments have to be planed, executed, analysed and documented.

*DIN EN ISO 9000:2005* defines validation as "confirmation, through the provision of objective evidence, that the requirements for a specific intended use or application have been fulfilled". Normalised methods are regarded as validated according to *DIN EN ISO*/*IEC 17025:2005* after their compilation. It should be distinguished between the first or *developmental validation* of a new test system and the so called *in house validation* of existing test systems in a specific laboratory environment. *In house validations* should be done by the introduction of a new test (first time validation) and repeated in defined time intervals. Furthermore, ring trails are often carried out to assess the precision (repeatability and reproducebility) and to define common quality standards between different laboratories. Ring trails also allow benchmarking between laboratories.

There is a need for clearly defined test and reference substances in high quality for validation procedures.

It should be emphasized that there are currently no official calibration standards or reference materials available especially for the quantification of proteins and nucleic acids (Ellison SL, English CA, Burns MJ, Keer JT: Routes to improving the reliability of low level DNA analysis using real-time PCR. BMC Biotechnol 6: 33, 2006). A further problem arises from the pipetting error during the dilution of DNA quantification standards. In addition, long time stability of standards which is sometimes difficult in the case of natural polymers like nucleic acids and proteins must be guaranteed to achieve reproducibility. This is especially important in the case of low amounts of these substances. Furthermore, the manufacturing of reference substances has to be carried out under strict quality control to avoid secondary contaminations.

The Directive 2004/9/EC of THE EUROPEAN PARLIAMENT and of THE COUNCIL of 11 February 2004 on the inspection and verification of good laboratory practice (GLP) (Official Journal of the European Union, Vol. L 50, pp. 28-43, 20 February 2004) states in *Part B* (Revised guidance for the conduct of test facility inspections and study audits) following inspection and audit criteria for test systems and test and reference substances: "Test systems Purpose: to determine whether adequate procedures exist for the handling and control of the variety of test systems required by the studies undertaken in the facility, for example, chemical and physical systems, cellular and microbic systems, plants or animals (...). Test and reference substances Purpose: to determine whether the test facility has procedures designed (i) to ensure that the identity, potency, quantity and composition of test and reference substances are in accordance with their specifications, and (ii) to properly receive and store test and reference substances.....".

According to *DIN EN ISO 9000:2005* certified reference material (CRM) is a reference material, accompanied by a certificate, one or more of whose property values are certified by a procedure that establishes metrological traceability to an accurate realization of the unit in which property values are expressed, and for which each certified value is accompanied by an uncertainty at a stated level of confidence.

Processing of the analytical results into DNA profiles also needs to be carefully controlled, and strict guidelines for the identification of alleles have to be developed and followed, to take account of such things as stutters, artefacts, peak size and morphology, variations in peak ratios, and so on. Three independent scientists, or two scientists and an expert system, are currently used in the UK to confirm the accurate designation of DNA profiles.

Hence, there is need for a kit and methods for testing amplification methods in particular in the forensic field. The present invention addresses this need.

### SUMMARY OF THE INVENTION

The invention relates to a kit comprising, (a) a first group of containers, comprising two or more containers with human genomic DNA, wherein the human genomic DNA in each of the two or more containers is of different human origin and the amount of DNA in the container is defined, (b) a second group of containers, comprising two or more containers with human genomic DNA, wherein the concentration of the DNA varies between the containers and the concentration of the DNA in the container is defined and (c) a third group of containers, comprising two or more containers with human genomic DNA, wherein the human genomic DNA in each container is a mixture of genomic DNA and is from at least two different individuals.

The invention relates to methods for producing a kit according to the invention as well as use of the kit according to the invention.

As used herein, a kit is a packaged combination optionally including instructions for use of the combination and/or other reactions and components for such use.

Herein, a container is a vessel made of any useful material for carrying DNA, such as a plastic vessel or a glass vessel, a capillary, or a vial, or a vial in a microtiter plate. The vessel is ideally closable.

Herein, human genomic DNA is a sample comprising at least a fraction of human genomic DNA, wherein at least one copy of each human chromosome is present, *i*.*e* in a male individual the male set and for a female individual the female set.

Herein, a male individual is an individual or a cell line that carries a full male chromosome set.

Herein, a locus or genetic locus is a specific position on a chromosome.

Herein, a female individual is an individual or a cell line that carries a full female chromosome set.

### DETAILED DESCRIPTION OF THE INVENTION

The present invention relates to a kit comprising, (a) a first group of containers, comprising two or more containers with human genomic DNA, wherein the human genomic DNA in each of the two or more containers is of different human origin and the amount of DNA in the container is defined, (b) a second group of containers, comprising two or more containers with human genomic DNA, wherein the concentration of the DNA varies between the containers and the concentration of the DNA in the container is defined and (c) a third group of containers, comprising two or more containers with human genomic DNA, wherein the human genomic DNA in each container is a mixture of genomic DNA and is from at least two different individuals.

This invention addresses many urgent needs in, e.g. forensic laboratories.

It addresses the need to be able to define test sensitivity, i.e. the amount of DNA that can be detected reliably.

It addresses the need to be able to define the amount of DNA that the laboratory can detect reliably in mixture wherein there is a presence of another DNA.

It addresses the need to b able to define the reproducibility with which the laboratory can detect gender specific loci.

It addresses the need to be able to ensure that only human DNA is amplified an no false amplification occurs do the presence of non-human DNA.

It addresses the need to be able to calibrate the reproducibility of, e.g. signal intensity, laboratory equipment, chemicals or enzymes.

It addresses the need to be able to understand the influence of certain PCR inhibitors.

The invention makes it possible to establish a Germany wide, Europe wide, or even global quality standard for forensic amplification. In one embodiment of the invention, the kit according to the invention is applied in a series of events over time and the data outcome is collected. This enables both governmental control of forensic laboratories, but more importantly makes it possible to identify grave events in laboratory practice which otherwise would not be detectable immediately. Thus, if for example a new employee does not maintain the same high laboratory standards previously maintained data from the test kit will identify this reduction in quality when the data is compared to previous data sets. In one embodiment the data is entered into laboratory management system (LIMS) in a further embodiment the data is kept in or sent to a central data repository.

One of the important aspects of the present invention is, that although some elements of the invention may be commercially available, such as genomic DNA, it is for the first time possible to address all of the above needs making use of one kit, with in a preferred embodiment one source of water and one source of many other agents in the kit.

DNA controls are per se known in the art. However, the isolation of DNA is error prone. Such controls may comprise contamination. Thus, in one embodiment the kit according to the invention is produced under conditions which are ISO 9001 and accredited to the NAMAS M10 standard which is ISO Guide 25 compliant. In a preferred embodiment the test kit is produced under ISO/IEC 17025 conditions.

Ideally the standard is as follows. ISO/IEC 17025:2005 specifies the general requirements for the competence to carry out tests and/or calibrations, including sampling. It covers testing and calibration performed using standard methods, non-standard methods, and laboratory-developed methods. It is applicable to all organizations performing tests and/or calibrations. These include, for example, first-, second- and third-party laboratories, and laboratories where testing and/or calibration forms part of inspection and product certification. ISO/IEC 17025:2005 is applicable to all laboratories regardless of the number of personnel or the extent of the scope of testing and/or calibration activities. When a laboratory does not undertake one or more of the activities covered by ISO/IEC 17025:2005, such as sampling and the design/development of new methods, the requirements of those clauses do not apply. ISO/IEC 17025:2005 is for use by laboratories in developing their management system for quality, administrative and technical operations. Laboratory customers, regulatory authorities and accreditation bodies may also use it in confirming or recognizing the competence of laboratories. ISO/IEC 17025:2005 is not intended to be used as the basis for certification of laboratories.

Compliance with regulatory and safety requirements on the operation of laboratories is not covered by ISO/IEC 17025:2005.

DNA quantification is very critical for the validation of test sensitivity, especially for quantitative methods. The quality and purity of genomic and plasmid DNA are tested by spectrophotometer and electrophoresis. Preferably, DNA is stored in buffered solutions, the most preferred is TE buffer (10 mM Tris/HCl, pH 8.0; 1 mM EDTA). Traditionally, DNA concentration has been determined by measuring absorbance of the sample at 260 nm (Mueller H, Ziegler B, Schweizer B. UV-VIS spectrometric methods for qualitative and quantitative analysis of nucleic acids. Int Spectoscopy Laboratory 4, pp. 4-11, 1993). Disadvantages of this method include its poor sensitivity and the impossibility to distinguish between single-stranded and double-stranded DNA and RNA. Furthermore, UV spectra are not very specific, and many other substances display absorbance in the wavelength range between 200 nm and 400 nm. Some buffer substances, matrix impurities (e.g. phenol from DNA extraction procedures) and polymers like proteins interfere at 260 nm. In practise, the absorbance ratio A₂₆₀/A₂₈₀ (absorbance at a wavelength of 260 nm and 280 nm) is used to measure impurities of DNA solutions. The quotient should be between 1.7 and 2.0 (detected in TE-buffer, pH 7.0 - 8.0). Contamination by RNA (quotient >2.0), proteins, polysaccharides and proteoglycans and matrix impurities like phenol (quotient <1.7) has been effectively eliminated. Sometimes a baseline correction for unspecific absorbance, most commonly at 320 nm, is used [A₂₆₀-A₂₃₀; (A₂₆₀-A₂₃₀)/ (A₂₈₀-A₂₃₀). The following formulas are used for UV-VIS quantification of nucleic acids:
- Double stranded (ds)DNA: 1 A ₂₀₀ = 50 µg/mL
- Single stranded (ss)DNA: 1 A ₂₆₀ = 33 µg/mL
- RNA: 1 A₂₆₀ = 40 µg/mL
- Sometimes, the Warburg-Christian-formula is used for DNA quantification to compensate for proteins (Warburg O, Christian W. Isolation and crystallization of enolase. Biochem. Z. 310, pp. 384-421, 1942): c_{DNA} = 62.9 x A₂₆₀ -36.0 A₂₈₀ [µg/mL].

There are a variety of different DNA and RNA quantification methods and kits available. PicoGreen^{®} (Molecular Probes, Invitrogen Corp., Carsbad, CA, USA) is a fluorescent dye that undergoes a dramatic fluorescence enhancement upon binding to dsDNA that can be measured using a microplate fluorometer. Other fluorescent dyes are known which selectively bind to dsDNA and RNA (Rengarajan K, Cristol SM, Mehta M, Nickerson JM. Quantifying DNA concentrations using fluorometry: a comparison of fluorophores. Mol Vis, 8, pp. 416-421, 2002). Quantitative real-time PCR assays based on fluorescent dyes or fluorescently labelled probes are very sensitive DNA quantification methods (US patent no. US 5,432,272 and European Patent Nos. EP 512,334; EP 543,942; EP 745,690; EP 1,088,102). Another method is based on the chemo luminescence measurement of pyrophosphate which is released from a polymerase reaction (Alu Quant™ Human DNA Quantitation System (Promega, Madison, WI, USA). European Patent No. EP 1,108,066 discribes an HPLC-based quantification of nucleic acids. The common technical feature of these techniques is that all of them need an external calibration curve which is based on a dilution series of a DNA sample which originally has been quantified by UV-VIS spectroscopy.

For producing the kit according to the invention it is necessary to stabilise the DNA and the other component. This is done with trehalose, saccharose, glycerin, polymers such as ficoll, dextran, polyvinylpyrolidon, polyacrylamide, other substances such as prolin, ectoin, betain, trimethylammoniumchlorid. Also a mixture of a selection of these compounds with optimized concentrations may be used.

Other reagents which are ideally added may be anti-microbial agents or sodium azide.

Human genomic DNA is a sample comprising at least a fraction of human genomic DNA. Said sample may additionally comprise other biomolecules such as proteins, mitochondrial DNA or lipids but is ideally free of such biomolecules. In a preferred embodiment the human genomic DNA is isolated from a tissues or a cell line and is in soluble form in water or in a buffer system. However the human genomic DNA may also be lyophilized in the presence of stabilizing reagents or air dried.

It is preferred that the DNA is isolated from cell lines (e.g. human cell line K562), reference material deposited at cell culture collections (e.g. American Type Culture Collections, ATCC; German Collection of Microorganisms and Cell Cultures, DSMZ; The Coriell Cell Repositories at Coriell Institute for Medical Research, Camden, NJ, USA).

In some embodiments recombinant DNA is used for positive controls. This is especially important in the case of rare mutations for which no stable cell line exists (e.g. specific DNA variations with are associated or responsible for cancer). Recombinant DNA inserts may be derived from natural sources or deduced from published sequences and chemically synthesized. The DNA may be cloned in different vector systems like bacteriophages, plasmids, phagemids, BACs, YACs and PACs. Genomic DNA from immortalized cell-lines like somatic cell fusion hybrids (monoclonal hybridoma cells, cell chimeras) and radiation hybrid clones is also possible. In some embodiments it is important to ensure that several target sequences are present in equal copy numbers or defined copy ratios. This can be achieved according to the invention by cloning these DNA fragments in the same vector "*in cis*" by linking the sequences in single copy series or as DNA concatemers.

Analytical specificity is the ability of an assay to exclusively identify a target substance or organism rather than similar but different substances in a sample or specimen (Saah AJ, Hoover DR. "Sensitivity" and "specificity" reconsidered: The meaning of these terms in analytical and diagnostic settings. Ann Intern Med, 126, pp. 91-94, 1997).

In the case of forensic DNA amplification assays a representative set of DNA sources of non human origin should be tested to exclude and/or document cross reactivity (see table 1). This may include DNA from phylogenetically closely related species like primates as well as farm animals and pets which life in closeness to men. A minimum of 2 ng of total DNA amount of these organisms should be applied per PCR to validate forensic stain kits. Furthermore, the validation schema should include representatives of human microbial microflora. Dethlefsen et al. (Dethlefsen L, McFall-Ngai M, Reiman DA. An ecological and evolutionary perspective on human-microbe mutualism and disease. Nature 449, pp. 811-818, 2007), Turnbaugh et al. (Turnbaugh PJ, Ley RE, Hamady M, Fraser-Liggett CM, Knight R, Gordon JI. The human microbiome project. Nature. 449, pp. 804-810, 2007) and references therein teach the state of the art and further perspectives of the ecology of human microbial flora. Non-steril human micro-environments which are sources of forensic DNA samples are skin, mouth (e.g. buccal swabs, sputum), oesophagus, stomach, vagina, gut, colon and faeces. Table 1 includes some major microbial species which are frequently found within samples prepared from healthy persons.

**Table 1: Collection of species for validation of the specificity of human and forensic diagnostic test systems.**

| **Trivial (common) name** | **Scientific name** | **Source or location** |
|---|---|---|
| **horse** | ***Equus caballus*** | **farm animal, meat** |
| **cattle** | ***Bos taurus*** | **farm animal, meat** |
| **cat** | ***Felis catus*** | **pet, laboratory animal** |
| **dog** | ***Canis lupus familiaris*** | **pet, laboratory animal** |
| **mouse** | ***Mus musculus*** | **pet, laboratory animal** |
| **rat** | ***Rattus norvegicus*** | **pet, laboratory animal** |
| **rabbit** | ***Oryctolagus cuniculus*** | **farm animal, meat** |
| **sheep** | ***Ovis aries*** | **farm animal, meat** |
| **golden (Syrian) hamster** | ***Mesocricetus auratus*** | **pet, laboratory animal** |
| **guinea pig** | ***Cavia aperea*** | **pet, meat, laboratory animal** |
| **pig** | ***Sus scrofa*** | **farm animal, meat** |
| **orangutan** | ***Pongo pygmaeus*** | **non-human Ape** |
| **chimpanzee** | ***Pan troglodytes*** | **non-human Ape** |
| **Western gorilla** | ***Gorilla gorilla*** | **non-human Ape** |
| **bonobo** | ***Pan paniscus*** | **non-human Ape** |
| **chicken** | ***Gallus gallus*** | **farm animal, meat** |
| | ***Acinetobacter johnsonii*** | **human normal skin** |
| | ***Aspergillus niger*** | **soil, air** |
| | ***Bacillus subtilis*** | **soil, air** |
| | ***Bacteroides fragilis*** | **human stool, distal gut** |
| | ***Bifidobacterium longum*** | **human stool, distal gut** |
| | ***Candida albicans*** | **human skin, vagina** |
| | ***Corynebacterium tuberculostearicum*** | **human normal skin** |
| | ***Corynebacterium coyleiae*** | **human normal vaginal microflora** |
| | ***Corynebacterium singulare*** | **human normal skin** |
| | ***Enterococcus faecalis*** | **human normal vaginal, faeces** |
| | ***Escherichia coli*** | **human skin, stool** |
| | ***Finegoldia* AB109769** | **human normal skin** |
| | ***Gemella haemolysans*** | **human normal oral mricroflora** |
| | ***Granulicatelia adiacens*** | **human normal oral mricroflora** |
| | ***Lactobacillus crispatus*** | **human normal vaginal microflora** |
| | ***Lactobacillus panis*** | **human normal vaginal microflora** |
| | ***Lactobacillus rhamnosus*** | **human intestine, stool and dairy products** |
| | ***Methanobrevibacter smithii*** | **human stool, distal gut** |
| | ***Neisseria gonorrhoe*** | **human skin** |
| | ***Neisseria spp.*** | **human normal oral mricroflora** |
| | ***Peptostreptococcus harei*** | **human normal vaginal microflora** |
| | ***Peptostreptococcus vaginalis*** | **human normal vaginal microflora** |
| | ***Propionibacterium acnes*** | **human normal skin** |
| | ***Pseudomonas stuzeri*** | **human normal skin** |
| | ***Saccharomyces cerevisiae*** | **human skin** |
| | ***Staphylococcus aureus*** | **human skin, oral flora** |
| | ***Staphylococcus epidermitis*** | **human normal vaginal and skin microflora** |
| | ***Streptococcus mitis*** | **human normal oral and skin microflora** |
| | ***Veillionella pravulum*** | **human normal oral mricroflora** |

Microbial type strains deposited at microbial strain collections (e. g. American Type Culture Collections, ATCC; German Collection of Microorganisms and Cell Cultures, DSMZ) or pre-characterized field isolates may be used as sources for DNA standard preparation. Due to the fact that microbial genomes are 100 (yeast) to 1000 fold (bacteria) smaller than mammalian once less DNA amounts are needed for validation studies. Typically, DNA amounts which are equivalent to at least 10⁵ genome copies per PCR are sufficient. In some embodiments mixtures of defined DNA samples from microbials are used within one reaction vessel to safe reagents.

In other embodiments of the invention more complex sample matrices are used as DNA sources for the validation of genotyping test specificity. Examples are PCR or qPCR assays for the detection of pathogen microorganisms. In the case of pathogens which infect the mammalian intestine DNA-extracts from the faeces of healthy humans and animals are the ideal sources. These extracts contain a natural mixture of DNA from the host cells (shedded epithelia and blood cells), the normal gut microflora and indigested food material (e.g. DNA from plant fibers in case of ruminant animals). Sometimes complex DNA-extracts are of especial value because it is known that many microbes from gut or environmental samples which may cross-react with the analytical test system are not culturable and, thus, are not characterized in detail. Other complex DNA extracts are possible for PCR tests dedicated to quality assessment of food or environmental samples.

Inhibitors of PCR are a serious problem in forensic case work. Although a variety of efficient DNA extraction and purification kits is commercially available which efficiently remove proteinous inhibitors (e.g. heparin) some water-soluble compounds may overcome the purification steps. Furthermore, analysis of forensic stains with low amounts of DNA requires the concentration of DNA extracts from a huge amount of sample material. Wilson gives an overview of inhibitors (Wilson IG. Inhibition and Facilitation of Nucleic Acid Amplification, Appl Environ Microbiol 63, pp. 3741-3751, 1997). Water-soluble model substances can be used as standards for validation purposes. The following compounds are preferred according to the invention: A potent mixture of inhibitors which is extracted from soil or other environmental sample is humic acid (CAS registry number 1415-93-6). Hemin (chloro [3,7,12,17-tetramethyl-8,13-divinylporphyrin-2,18-dipropanoato(2-)] iron(III)) (CAS registry number 14875-96-8 ) or haematin (CAS registry number 15489-90-4) are degradation products of haemoglobin derived from blood samples. Indigo carmine (CAS registry number 860-22-0) resembles the colour of cotton jeans. Bile salts (equimolar mixture of sodium salts of cholic acid and desoxycholic acid) are water soluble extracts of faeces. Other examples are known to those who are skilled in the art.

Extracts of natural and synthetic products or mixtures thereof are attractive inhibitors, too. For example hot aqueous extracts (boiling in deionized water for 20 min, supernatant after centrifugation at 12,000 x g for 10 min at room temperature) of cigarette ash or newspapers (inhibition by printing ink) can be standardized for validation purposes.

DNA derived from forensic stains (e. g. site of crime, dead bodies, skeletons, common graves) or archaeological samples is often highly degraded. Fragment sizes smaller than 500 bp, 300 bp, 200 bp, 150 bp and 100 bp negatively affect the amplification of multiplex PCR assays. Thus, validation standards with DNA which is degraded under defined and reproducible conditions and which is clearly characterized and documented are highly recommended.

Purified DNA can be degraded by physical, chemical and enzymatic methods. However, it is important to distinguish between DNA fragmentation which is intended according to the invention and DNA inactivation (e. g. with regard to functional test like PCR or DNA cloning). Many chemical methods, especially those using oxidative reagents, suffer from the disadvantage that their effect is not selective enough. Chemical derivatisation of nucleotide bases which prevent PCR primer binding and elongation almost dominate over fragmentation by cleavage of the phosphordiester backbone of double stranded DNA (dsDNA).

One preferred enzymatic method according to the invention is the use of the dsDNA-specific endonuclease deoxyribonuclease (DNAse) I which acts as a dsDNA-specific phosphodiesterase and produces in the presence of manganese ions preferentially double strand breaks in dsDNA (Sambrook J, Fritsche EF, Maniatis T. Molecular cloning, A laboratory manual. 2nd edition, Cold Spring Harbor Laboratory Press, 1989). However, it should be noted that the reproducibility of this method outmost depends on the source, concentration and quality of DNA and the enzyme DNAse I. Due to this facts, time consuming kinetic pre-experiments are always required to define the proper reaction conditions from batch to batch.

With regard to physical methods DNA shearing based on hydrodynamic point-sink techniques is most preferred. The rational behind these techniques is that a more or less compressed DNA solution in water or buffer is expanded through a very narrow valve or orifice. This hydrodynamic effect causes a shearing force on DNA molecules which is unbiased, independent of the DNA source and highly reproducible. One commercially available apparatus is the so called "French pressure cell press" (French CS, Milner HW. Disintegration of bacteria and small particles by highpressure extrusion, pp. 64-67. In: Colowick SP, Kaplan NO (ed.), Methods in enzymology, vol. 1. Academic Press Inc., New York, 1955). Recently, Thorstenson et al. (Thorstenson YR, Hunicke-Smith SP, Oefner PJ, Davis RW. An automated hydrodynamic process for controlled, unbiased DNA shearing. Genome Res, 8, pp. 848-855, 1998.) introduced a continuous setup based on high precision narrow laser-drilled ruby jewel orifices and a syringe pump system (or HPLC-pump). Comparable instrumental setups are most preferred. Ultrasonic shearing is also recommended but gives less reproducible results.

In some embodiments, combinations of physical and enzymatic methods are preferred (Bender K, Farfán MJ, Schneider PM. Preparation of degraded human DNA under controlled conditions. Forensic Sci Int, 139, pp. 135-140, 2004).

DNA size fractionation has to be done after the shearing experiment if the DNA fragment size distribution is not ideal for validation purposes. This can be done by preparative agarose gel electrophoresis, ion pair reverse phase high performance liquid chromatography (HPLC), gel filtration, saccharose or caesium chloride density ultra centrifugation (Sambrook J, Fritsche EF, Maniatis T. Molecular cloning. A laboratory manual. 2nd edition, Cold Spring Harbor Laboratory Press, 1989) or fractionized DNA precipitation in the presence of polyethylene glycol (Lis JT. Fractionation of DNA fragments by polyethylene glycol induced precipitation. Methods Enzymol. 65, pp. 347-353, 1980). Quality and quantity of the degraded DNA standard is finally analyzed by gel electrophoresis and UV/VIS spectrometry.

In a preferred embodiment the first group of containers comprises two or more containers each comprising human genomic DNA, wherein at least one DNA is from a male individual and one DNA is from a female individual. It is important that the respective individual has a normal chromosomal set including all chromosomes.

It is preferred that the human genomic DNA in the second group of containers has a quantity between 5 pg and 10 ng. The determination of the precise concentration is one important aspect of the present invention. The concentration may be determined by any of the methods outlined above.

It is preferred that the mixture of DNAs in the third group of containers has a ratio of between 1 to 3500 and 3500 to 1, wherein the larger fraction has a quantity between 200 pg and 900 ng.

It is preferred that the mixture of DNAs in the third group of containers has a ratio of between 1 to 100 and 100 to 1, wherein the larger fraction has a quantity between 50 pg and 2 ng.

It is preferred that the mixture of DNAs in the third group of containers has a ratio of between 1 to 50 and 50 to 1, wherein the larger fraction has a quantity between 200 pg and 800 pg.

It is further preferred that the third group of containers comprises at least one container which carries an equal amount of said two genomic DNAs. Ideally this equal amount is an equal amount with respect to a given locus to be amplified. The container should ideally thus contain, e.g. at least one copy of a given locus from said male individual and one copy of said given locus from a female individual.

In a preferred embodiment the kit according to the invention comprises a fourth group of containers and this group comprises containers with human genomic DNA and an amplification reaction inhibitor. Ideally this is a polymerase chain reaction inhibitor and it inhibits the polymerase.
Inhibitors are a serious problem in forensic case work. Wilson gives an overview (Inhibition and Facilitation of Nucleic Acid Amplification, Ian G. Wilson, Applied and Environmental Microbiology, Oct. 1997, pp. 3741-3751)

In a preferred embodiment the polymerase chain reaction inhibitor is selected from the group comprising humic acid, hemin (chloro[3,7,12,17-tetramethyl-8,13-divinylporphyrin-2,18-dipropanoato(2-)]iron(III)), indigo carmine and bile salts (equimolar mixture of sodium salts of cholic acid and desoxycholic acid). Further inhibitors may be selected from Wilson (1997) (Wilson IG. Inhibition and Facilitation of Nucleic Acid Amplification, Appl. Environ. Microbiol. 63, pp. 3741-3751, 1997).

Preferable the fourth group of containers comprises at least one container with human genomic DNA from a male individual and one container with human genomic DNA from a female individual. Thus, the kit comprises at least one container with human genomic DNA from a male individual as well as said inhibitor and additionally a further container with human genomic DNA from a female individual as well as said inhibitor.

Preferably the kit additionally comprises a fifth group of containers comprising at least one container with a first non-human genomic DNA sample. Non human DNA samples enable testing of analytical specificity. This test is important because non-human DNA may cause a problem in testing but may also be used in testing.

While the use of human DNA in criminal cases is widespread, nonhuman DNA found at a crime scene can be also be important in helping to solve a case. Below are decisions where plant DNA, animal DNA and viral DNA have been found to be admissible in criminal cases.

PCR based RAPD technology for plant DNA was ruled admissible in a murder case (State v. Bogan, 183 Ariz. 506, 905 P.2d 515 (Ariz.App. Div. 1, Apr 11, 1995) (NO. 1CA-CR93-0453)

PCR testing on swine DNA was ruled admissible in a criminal case (U.S. v. Boswell,--- F.3d ----, 2001 WL 1223128 (8th Cir. (lowa), Oct 16, 2001) (NO. 00-4005)).

DNA testing of HIV virus was ruled admissible in an attempted murder case (State v. Schmidt, 97-249 (La.App. 3 Cir. 7/29/97), 699 So.2d 448 (La.App. 3 Cir., Jul 29, 1997) (NO. K97-249)).

DNA testing of HIV virus was ruled admissible in an attempted murder case (State v. Schmidt, 99-1412 (La.App. 3 Cir. 7/26/00), 771 So.2d 131 (La.App. 3 Cir., Jul 26, 2000) (NO. 99-1412)).

DNA testing of cat hair was ruled admissible in a murder case (Beamish v. Her Majesty The Queen, In the Supreme Court-Appeal Division for the Province of Prince Edward Island, Docket # AD-0693 & 7/22/99).

Results from DNA testing of a deer was used to obtain a search warrant (State v. Demers , 167 Vt. 349, 707 A.2d 276 (Vt., Dec 26, 1997) (NO. 96-452)).

Dog DNA testing and a canine database was ruled admissible in a double homicide trial Washington (v. Tuilefano & Lealuaialii , Superior Court of Washington for King County, No. 97-1-01391-3SEA & 96-1-08245-9SEA, 1/5/98)).

The non-human genomic DNA sample is preferably selected from the group of dog, cat, cow, horse, sheep, chicken, gorilla, orang-utan, chimpanzee, macaque, *E. coli*, and *Candida albicans*. Other non-human DNA samples are also preferred (see table 1).

Also the non-human DNA may be present in the form of dilution series.

Ideally the kit according to the invention comprises a sixth group of containers comprising at least one container with a first human genomic DNA sample which is degraded. A number of studies have demonstrated that successful analysis of degraded DNA specimens from mass disasters or forensic evidence improves with smaller sized polymerase chain reaction (PCR) products. To understand the factors that influence the successful amplification of STR systems when using a limited amount of highly degraded template DNA, a source of standardized degraded DNA is helpful. It is important to assess to which extent a laboratory is able to work with degraded DNA. To this extent of course it is helpful to define the threshold precisely.

Degraded DNA may be prepared by many different ways. The preferred way herein is shearing with a hydrodynamic point-sink technique. Bender et al. discloses another way (Bender et al, 2004, Preparation of degraded human DNA under controlled conditions, Forensic Science International 139 (2004) 135-140). See also methods outlined above.

Thus, in one embodiment the kit according to the invention comprises a sixth group which comprises more than one container, wherein each container comprises a human genomic DNA sample and wherein each genomic DNA sample has a different state of degradation. Such a series allows the laboratory exactly to determine the extant to which the laboratory is able to work with degraded DNA.

Ideally the kit additionally comprises a seventh group of containers comprising at least one container with a first human genomic DNA sample as well as a second human genomic DNA sample, wherein the second human genomic DNA is from a different individual than the first sample and the second sample is degraded.

The kit may additionally comprise an eighth group of containers comprising at least one container with no DNA but either water or a buffer or another solution which may be added to an amplification reaction.

The kit may additionally comprise a ninth group of containers with a mixture of male DNA and female DNA wherein the containers ideally have the same amount of male DNA and differing amount female DNA. This test is very important because it is thus possible to test Y-specific amplification.

A microtiter plate or microplate is a flat plate with multiple "wells" used as small test tubes. The microplate has become a standard tool in analytical research and clinical diagnostic testing laboratories. A very common usage is in the enzyme-linked immunosorbent assay (ELISA). A microtiter plate typically has 6, 24, 96, 384 or even 1536 sample wells arranged in a 2:3 rectangular matrix. Some microplates have even been manufactured with 3456 or even 9600 wells. Each well of a microplate typically holds somewhere between a few to a few hundred microliters of liquid.

The earliest microplate was created in 1951 by a Hungarian, Dr. G. Takatsky, who machined 6 rows of 12 "wells" in Lucite. However, common usage of the microplate began in the late 1950s when John Liner in USA had introduced a molded version. By 1990 there were more than 15 companies producing a wide range of microplates with different features. It was estimated that 125 million microplates were used in 2000 alone.

In 1996, the Society for Biomolecular Sciences (SBS) began an initiative to create a standard definition of a microtiter plate. A series of standards was completed in 2003 and published by the American National Standards Institute (ANSI) on behalf of the SBS. The standards govern various characteristics of a microtiter plate including well dimensions (e.g. diameter, spacing and depth) as well as plate properties (e.g. dimensions and rigidity).

A number of companies have developed robots to specifically handle SBS microplates. These robots may be liquid handlers which aspirate or dispense liquid samples from and to these plates, or "plate movers" which transport them between instruments.

Instrument companies have designed plate readers which can detect specific biological, chemical or physical events in samples stored in these plates.

Method of producing a test kit according to the invention wherein the DNA is dispensed into the containers, in soluble form, and wherein the dispensing is performed under clean room conditions. Preferably kit production occurs in a room with at least US FED STD 209E cleanroom standard class 100 which is compliant to ISO 14644 clean room standard class 5.

Concerning the clean room quality it is preferred that the air quality has grade of maximal 100,000 particle ≥0.1 µm per m³, 23,700 particle ≥0.2 µm per m³, 10,200 particle ≥0.3 µm per m³, 3,520 particle ≥0.5 µm per m³, 832 particle ≥1.0 µm per m³, 10,200 particle ≥0.3 µm per m³ and 29 particle ≥5.0 µm per m³ according to US FED STD 209E cleanroom standard class 100 which is compliant to ISO 14644 cleanroom standard class 5. The air entering the area of manufacturing from outside is filtered to exclude dust, and the air inside is constantly recirculated through high efficiency particulate air (HEPA) or ultra low penetration air (ULPA) filters.

In the case of RNA- or DNA-amplification standards, specific operation arrangements have to be taken during the manufacturing process to prevent contaminations with exogenous nucleic acids. For example, aqueous hypochlorite solutions (bleach, at least 0.6 %) or other cleaning and/or sterilization solutions based on oxidative reagents like peroxides or peroxyacetic acid are used to decontaminate surfaces and instruments. Air filters with charcoal are applicable to absorb traces of free DNA bound to aerosols or particles.

Ultra-clean, sterilized and certified plastic materials are preferred for reaction vessels (e.g. tubes, strips, microtiter plates), sealings (caps or folia) and pipet tips. Sterilization may be performed by radiation in accordance to ANSI/AAMI/ISO 11137 (Association for the Advancement of Medical Instrumentation/American National Standards Insitute/ International Organization for Standardization). Certification means that specific assay are performed to guarantee the absence of interfering RNA, DNA or proteins, the absence of DNAses, RNAses and/or proteases. In addition, the performance of the diagnostic assay (PCR, ELISA etc.) should be tested in advance to exclude the presence of inhibitors derived from the manufacturing process of the containers or parts of it.

Glass surfaces are known to adsorb especially DNA and, thus, should be avoided especially for quantitative DNA applications. This is also essential if low amounts of DNA are applied. Unspecific absorption of other analytes (e.g. proteins, lipids, phospholipids, carbohydrates, lipopolysaccharides etc.) to surfaces is also known and may be considered in corresponding assays. Some manufacturers offer vessels, plates or pipet tips in so called "low retention" quality which is generated by proprietary production steps. Generally, the analyte volume should be kept as small as possible. Some real-time PCR instruments like the LightCycler^{®} 2.0 System offered by *Roche Diagnostics GmbH* (Mannheim) need glass capillaries, the surfaces of which should be blocked by bovine serum albumin or pretreated with specific silane solutions (Teo IA, Choi JW, Morlese J, Taylor G, Shaunak S. LightCycler qPCR optimisation for low copy number target DNA.J Immunol Methods. 2002, Vol. 270, pp. 119-133; Sambrook J, Fritsche EF, Maniatis T. Molecular cloning. A laboratory manual. 2nd edition, Cold Spring Harbor Laboratory Press, 1989). Furthermore, unrelated carrier DNA which gives no signal in the diagnostic test system of interest may be applied for surface passivation in DNA based assays. In the case of mammalian DNA test systems DNA from natural sources like from the *E. coli* bacteriophage lambda or from salmon sperm is preferred for this purpose. In some embodiments it is preferred to use DNA with an artificial sequence which is not found in nature. This can be bioinformatically designed by random combination of nucleotides which are then negatively selected using the *Blastn* algorithm *(Basic Local Alignment Search Tool nucleotides;* Altschul SF, Gish W, Miller W, Myers EW, Lipman DJ. Basic local alignment search tool. J Mol Biol, Vol 215, pp 403-410, 1990) against known natural occurring DNA, especially, against genomic DNA sequences derived from the target organism and closely related species.

In general, disposal pipet tips are preferred for liquid handling. Re-useable pipetting syringes or tips need to be rigorously cleaned between unrelated pipetting steps to avoid cross contamination. US Pat. No. 7,017,594 discloses the use of cold atmospheric plasma to decontaminate pipet tips just before there use. In addition, positive displacement pipettes offer advantages over air displacement pipettes with regard to cross contamination and precision. Air displacement pipettes should contain aerosol tight filters and are in the literature also referred to as filter tips. Automatic liquid handling with robot systems is highly recommended to improve reproducibility between parallel samples or batches of the kit.

The isolation of the genomic DNA that is filled into the containers is preferably performed under *DIN EN ISO*/*IEC 17025:2005* compliant conditions.

Deionised water used in the kit according to the condition preferably has a quality of "sterile and recommended for high performance liquid chromatography (HPLC)", more preferably the quality *aqua injectabilia* is used, especially in the case of immunological or cell based assays (free of pyrogenes like bacterial endotoxins, e.g. measured with the *Limulus* amebocyte lysate assay, US patent No. 4,322,217) and most preferably a quality which is analytically certified for PCR (absence of DNA, RNA and absence of nuclease activities) is applied in the case of RNA and DNA amplification tests. If not provided by the manufacturers, *in house* test must be performed to guarantee the quality.

Preferably kit production occurs in a room with at least US FED STD 209E cleanroom standard class 100 which is compliant to ISO 14644 cleanroom standard class 5.

The kit may be used for testing amplification reactions. Ideally such a reaction is polymerase chain reaction amplification. It may also be another form of amplification such as for example, rolling circle amplification (such as in Liu, et al., "Rolling circle DNA synthesis: Small circular oligonucleotides as efficient templates for DNA polymerases," J. Am. Chem. Soc. 118, pp. 1587-1594, 1996), isothermal amplification (such as in Walker, et al., "Strand displacement amplification-an isothermal, in vitro DNA amplification technique," Nucleic Acids Res. 20(7):1691-6 (1992)), ligase chain reaction (such as in Landegren, et al., "A Ligase-Mediated Gene Detection Technique," Science 241:1077-1080, 1988, or, in Wiedmann, et al., "Ligase Chain Reaction (LCR)-Overview and Applications," PCR Methods and Applications (Cold Spring Harbor Laboratory Press, Cold Spring Harbor Laboratory, NY, 1994) pp. S51-S64.)). Polymerase chain reaction amplification is preferred.

The kit may used to test forensic test kits based on the following polymorphic DNA types, short tandem repeats (STR), variable number tandem repeats, nucleotide sequence differences including insertion deletion polymorphisms, microsatellites, minisatellites, Y-STRs or any other polymorphic form of DNA. Likewise the kit may be used to test laboratories and/or chemicals and/or other reagents dealing and used for the analysis of "ancient DNA". Ancient DNA can be described as any DNA recovered from biological samples that have not been preserved specifically for later DNA analyses. Examples include the analysis of DNA recovered from archaeological and historical skeletal material, mummified tissues, archival collections of non-frozen medical specimens, preserved plant remains, ice and permafrost cores, and so on. Unlike modern genetic analyses, ancient DNA studies are characterised by low quality DNA. This places limits on what analyses can achieve. Furthermore, due to degradation of the DNA molecules, a process which correlates loosely with factors such as time, temperature and presence of free water, upper limits exist beyond which no DNA is deemed likely to survive. Current estimates suggest that in optimal environments, *i*.*e*. environments which are very cold, such as permafrost or ice, an upper limit of max 1 Million years exists.

The kit according to the invention may comprise such an amount of one or more of the DNAs according to the invention that multiple reactions may be performed or may comprise such an amount of sample DNA that only one reaction is ideally performed. The latter could be called a "one-time" kit.

Preferred components of the kit are shown in table 2.

**Table 2**

| | |
|---|---|
| **First group of containers** | **DNA from various individuals** |
| **Second group of containers** | **Dilution series of human genomic DNA** |
| **Third group of containers** | **Mixture of DNA from two, three or four individuals** |
| **Fourth group of containers** | **DNA with an amplification inhibitor in one defined or in different concentrations** |
| **Fifth group of containers** | **Several non-human DNA samples, separated or as a mixture** |
| **Sixth group of containers** | **Degraded DNA sample** |
| **Seventh group of containers** | **Degraded DNA mixed with no degraded DNA** |
| **Eighth group of containers** | **Water or other liquid without DNA** |
| **Ninth group of containers** | **DNA from male and female wherein the concentration of the female or male DNA differs** |
| **Tenth group of containers** | **DNA from one or several individuals which are pre characterized with regard to specific genetic mutations or polymorphisms (variations)** |
| **Eleventh group of containers** | **Recombinant DNA (plasmids, phagemids, BACs, PACs, YACs or other artificial constructs) which encode target sequences, specific pre chracterized mutations or polymorphisms, especially those which are rarely found in nature.** |
| **Twelfth group of containers** | **Mixtures of PCR amplificates or *in vitro* transcripts encoding target sequences** |

In one embodiment the kit according to the invention comprises at least 3 different groups of containers selected from those groups listen in table 2. In a further embodiment the kit according to the invention comprises at least 4 different groups of containers selected from those groups listen in table 2. In a further embodiment the kit according to the invention comprises at least 5 different groups of containers selected from those groups listen in table 2. In a further embodiment the kit according to the invention comprises at least 6 different groups of containers selected from those groups listen in table 2. In a further embodiment the kit according to the invention comprises at least 8 different groups of containers selected from those groups listen in table 2. In a further embodiment the kit according to the invention comprises at least 4 different groups of containers selected from those groups listen in table 2. In a further embodiment the kit according to the invention comprises the groups of containers listen in table 2.

Ideally the containers are suited for direct situation into an amplification device such as a PCR machine. In such a case a laboratory specific master mix, comprising a buffer deoxynucleotides, a polymerase and specific primers is added to the kit amplification is performed.

In an embodiment of the invention the invention relates to a method of analysing the quality of an amplification, wherein the following steps are applied,
a) an amplification is performed with at least the first set of containers, the second set of containers and the third set of containers,
b) the reaction products are analyzed and it is determined whether an amplification product is present.

Preferably a quality grade is established. Ideally, this is done for each series separately. Preferably thereafter an overall quality grade is calculated. The single and overall quality grades are sent to or kept in a database repository. The figures are tracked over time. After a certain amount of data points an average laboratory quality grade is established for this specific forensic laboratory. In a preferred embodiment an indicator is established, i.e. a threshold value, which will, when the laboratory produces a poor quality data check indicate to the laboratory that an out of the average poor quality result was produced. The same system can be applied to multiple laboratories, in order to, e.g. create a central repository for ensuring minimum standard quality in forensic amplification.

The invention also relates to a kit wherein the containers are wells in a micro titerplate and the plate has 6, 24, 96, 384 or even 1536 sample wells arranged in a 2:3 rectangular matrix.

A software solution may be useful as an expert system to document and control all steps of a validation procedure (planning, execution, analysis and documentation). In addition, all documents which are essential for the validation procedure can be generated and written by the software. Furthermore, all originally measured data are stored in a database which allows a comprehensive control of the complete procedure. The data analysis is clearly documented and an audit trail with date and time stamps (e. g. Guidance for Industry - computerized systems used in clinical trails, Food and Drug Administration USA, 1999 and Guidance for Industry - Part 11, Electronic Records; Electronic Signatures - Scope and Application, Food and Drug Administration USA, 2003) is implemented to ensure that all steps of data storage and processing are sure, reproducible and traceable. The software should be developed according to international standards (e.g. DIN EN ISO/IEC 12207; GOOD PRACTICES FOR COMPUTERISED SYSTEMS IN REGULATED "GXP" ENVIRONMENT, PIC/S 2003) to ensure a validateable software environment. Electronic signatures in compliance to national or international standards (e.g. German ,,Gesetz über Rahmenbedingungen für elektronische Signaturen SigG", 2001; Guidance for Industry - Part 11, Electronic Records; Electronic Signatures - Scope and Application, Food and Drug Administration USA, 2003) should be supported by the software.

In some embodiments sample tracking and electronic identification of the validation plates together with all analytical information is supported by barcode readers or RFiD (radio frequency identification) transponders.

Beside documentation and control of the validation procedure the software can be used for data analysis, too. For example, different statistical methods are accomplished with the software. Examples are the mean value and standard deviation for replicate sample measurements. Other statistical methods are variance, variation coefficient, chi-square test, F-test, t-test, outlier test according to Grubbs, Nalimov, Dixon or Hampel etc.

It is also possible to validate the whole intrinsic process of result assessment from the raw data to the diagnostic findings. One example is a kinship analysis with the complete bio-statistical interpretation (combined paternity index, probability of exclusion, probability of paternity etc.) according to guidelines like "Richtlinien für die Erstattung von Abstammungsgutachten", Bundesaerztekammer, 2002 (Hoppe J-D, Kurth R, Sewing K-F, Deutsches Aerzteblatt 99, pp. A665-A667, 2002). For this purpose, the software must include all reference data (population statistics for all genetic markers) and mathematic formula. An interface to specific analysis software [e.g. in the case of genotyping assays with DNA sequencing automates from Applied Biosystems (Foster City, USA): GeneMapper software (Applied Biosystems Inc., Foster City, USA) or Genoproof^{®} software, (Qualitype AG, Dresden, Germany)] or Laboratory Management Information Systems (LIMS) is advantageous.

In one embodiment the software is organized as an online, web-based version. The registered user deposits the validation data on the web-server and all functionalities of the software are available online. The data input may be possible via barcode or RFiD reader, too. Additionally, a benchmarking between different laboratories or the organisation of ring trails may be supported with this online software.

Another embodiment consists of a desktop solution of the software. The advantage of this embodiment is that there is no need for internet access. This version may include further information of the quality management (QM) according to ***DIN EN ISO*/*IEC 17025:2000,*** like the documentation of analytical instruments. All data are deposited in a database with research functionality. An anonymized benchmarking between laboratories may be possible via data files which are periodically provided.

Another embodiment of the software is a mixture of desktop and online version. All data are stored in a desktop database and may be part or linked to the Laboratory Management Information System (LIMS). In addition, the desktop version offers web-links for data exchange and the use of further web-based functionalities like the download of new validation protocols or the comparison of benchmark data.

The invention also relates to a kit comprising a first group of containers, comprising (i) two or more containers with at least two or more nucleic acids from a human pathogen either alone or as a mixture, (ii) two ore more containers with at least two or more nucleic acids from a human pathogen either alone or as mixture however, additionally comprising human genomic DNA, (iii) two or more containers with at least two or more nucleic acids from a human pathogen as well as human genomic DNA, additionally comprising a PCR inhibitor, such as hemin or EDTA, (iv) optionally one or more containers comprising one or more nucleic acids from a non-pathogenic organism.

### FIGURE CAPTIONS

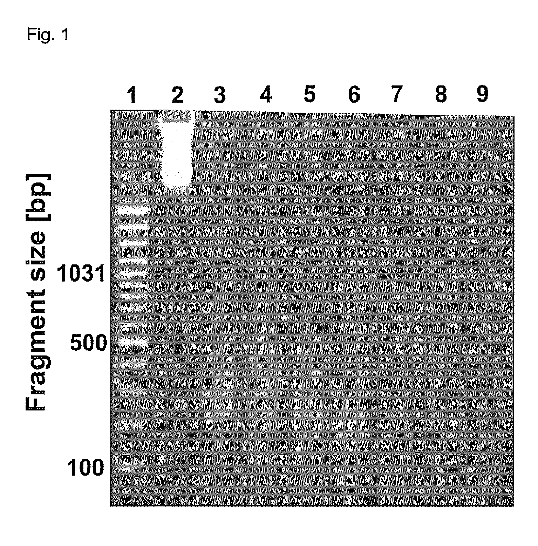

**Figure 1****: Praparation of degraded DNA by DNAse I digestion.** Shown is an ethidium bromide stained agarose gel with human genomic DNA which had been incubated with DNAse I according to the conditions explaint in Example 2 for 0 min (lane 2), 2 min (lane 3), 3 min (lane 4), 4 min (lane 5), 5 min (lane 6), 7 min (lane 7), 10 min (lane 8) and 15 min (lane 9). The *GeneRuler^{™} 100 bp DNA Ladder Plus* (lane 1; Fermentas Life Sciences, St. Leon-Rot , Germany) was used as a DNA length standard.

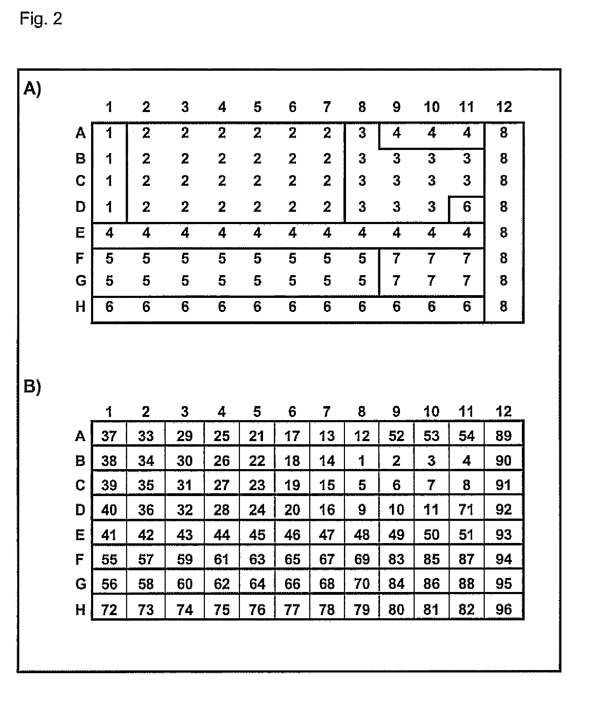

**Figure 2****: A kit according to the invention prepared with a microtiter plate. A)** Assignment of different groups of containers. (1) First group of containers: Test for species specificity. (2) Second group of containers: Analytical sensitivity. (3) Third group of containers: Blanc without DNA. (4) Fourth group of containers: Human DNA mixture study. (5) Fifth group of containers: Test of PCR inhibitors. (6) Sixth group of containers: Precision of allele calling. (7) Seventh group of containers: Degraded DNA. (8) Eighth group of containers: Forensic stain analysis. **B)** Pipetting schema. Details of the samples 1-96 are explained in the text (Example 3) and in table 5.

### EXAMPLES

### EXAMPLE 1

### Preparation of DNA, DNA quantification, serial DNA dilutions and DNA mixtures

Whole blood was collected from the veins of healthy human male and female volunteers with declared consent or from animals with compliance to guidelines of animal protection. A sterile 10 mL S-Monovette^{®} blood collecting device (Sarstedt, Nuembrecht, Germany) impregnated with citrate buffer was used to avoid blood coagulation. The blood was stored at room temperature until further use. All reagents and buffers were certified for the use in human molecular diagnostics (sterile, free of DNA, RNA and nucleases). Again, all work was done under the special clean room conditions outlined above. DNA from 9 mL "citrate blood" was isolated within one day after collection with a QIAamp^{®} DNA Blood Maxi Kit (Qiagen, Hilden, Germany) according the protocol of the manufacturer with following modifications. The DNA was eluted from the purification columns 3fold with 1 mL certified deionized water (Applied Biosystems/Ambion, Austin, TX, USA) and finally stored in 4 mL of a buffer consisting of 1 mM Tris/HCl (pH 8.0, measured at room temperature), 0.1 mM EDTA and 100 mM trehalose. This buffer, termed "TTE-buffer", was also used for dilutions and zero controls. Tenfold stock solutions of the buffer components and water were added to the DNA which had been eluted with 3 mL water to give a final volume of 4 mL. All buffer stock components except trehalose were certified for PCR and obtained from Applied Biosystems/Ambion (Austin, TX, USA). Trehalose (Sigma Aldrich Chemie GmbH, Munich, Germany) was dissolved in certified deionized water to give a 1 molar stock concentration. Quantitative real time PCR (qPCR) using the Quantifyler™ Human DNA quantification kit (Applied Biosystems, Foster City, CA, USA) and an *ABI Prism 7000 SDS* real time thermocycler (Applied Biosystems, Foster City, CA, USA) was applied to certify the absence of human DNA in the buffer and water.

Bacteria, yeasts and moulds were cultivated on agar plates according to standard methods (Atlas RM. Handbook of Microbiological Media, 3rd edition, CRC Press, Boca Raton, FL, USA, 2004; Dworkin M, Falkow S, Rosenberg E, Schleifer, K-H, Stackebrandt E. The Procaryotes. Vol. 1-7. 3rd edition, Springer-Verlag GmbH, Heidelberg, Germany). Sabouraud-Dextrose agar (Merck KGaA, Darmstadt, Germany) was used for yeasts and Potato-Dextrose agar (Merck KGaA, Darmstadt, Germany) for moulds.

Microbial cells or fungal conidia were scraped from a completely overgrown agar plate with a sterile spatula and resuspended in 5 mL lysis buffer [100 mM Tris/HCL, pH 8.0, 2 % (vol./vol.) Triton X-100, 1 % (wt./vol.) sodium dodecyl sulfate, 1 mM EDTA]. Aurintricarboxylic acid was added to a final concentration of 100 µM to inhibit nucleases which may be secreted by some bacteria and fungi. Lysozyme (1 mg/mL) was added to suspensions of some gram-positive bacteria to facilitate cell lysis. Yeast and mould cells were desintegrated by the addition of 2 g steril glass beads (0.45-0.55 mm diameter, Sigma Aldrich GmbH, Munich, Germany) and by shaking for 3 min with a *Precellys* 24 homogenisator (Peqlab Biotechnologie GmbH, Erlangen, Germany) at maximal speed setting. The cell lysates were then cleared by filtration (0.45 µm sterile filter) and/or centrifugation (18,000 x g, 15 min, at room temperature) and subjected to the QIAamp^{®} DNA Blood Maxi Kit (Qiagen, Hilden, Germany). DNA purification was then done according to the protocols of the manufacturer for bacteria and yeasts.

DNA quality and concentration were determined by agarose gel electrophoresis and UV/VIS-spectrometry as outlined above. An Eppendorf BioPhotometer^{®} (Eppendorf AG, Hamburg, Germany) was used for primary recording of DNA absorbancy at 260 nm (A₂₆₀). A₂₆₀-values were between 0.3 and 0.6 to guarantee high precision of the instrument. A baseline correction at 320 nm was performed (A₂₆₀-A₃₂₀). The quotient (A₂₆₀-A₂₃₀)/(A₂₈₀-A₂₃₀) was between 1.7 and 1.9. DNA concentrations were calculated based on the formula 1 A₂₆₀ = 50 µg/mL. Typically, DNA concentrations between 65 ng/µL and 180 ng/µL could be achieved. The quality of the DNA was further checked by agarose gel electrophoresis and ethidium bromide staining which gave a banding between app. 15-50 kb (length standards for agarose gele electrophoresis were lambda DNA and lambda DNA *Hind* III digest). Essential no RNA was detected.

DNA solutions, serial DNA dilutions and DNA mixtures were pipetted with a Corbett CAS-1200™ automated PCR setup robot and Tecan^{®} Genesis^{®} compatible 50 µL and/or 200 µL conductive steril filter tips (Corbett Biosciences, Mortlake, NSW, Australia). The robot possesses software for the calculation of dilution series and a specific mixing program to minimize pipetting errors. Alternatively, *Eppendorf Reference Pipettes* were used together with Eppendorf filter tips in "PCR clean" quality (sterile, certified DNA-free, RNase-free and pyrogen-free) (Eppendorf, Hamburg, Germany).

A mixture of human male and female DNA for the validation of a Y-chromosomal STR multiplex PCR amplification kit was made as follows: DNA from a male volunteer was diluted with TTE-buffer to 50 pg/µL. Female DNA was concentrated by ethanol precipitation (Sambrook J, Fritsche EF, Maniatis T. Molecular cloning. A laboratory manual. 2nd edition, Cold Spring Harbor Laboratory Press, 1989) and dissolved in TTE-buffer to a final concentration of 204.8 ng/µL. A serial dilution of the female DNA solution resulting in a geometric progression was prepared with the Corbett CAS-1200™ automated PCR setup robot (table 3) in 1.5 mL PCR-certified reaction tubes. Finally, 5 µL of the male DNA was pipetted to 13 new reaction tubes and 5 µL from each dilution of the female DNA, respectively, was added to the male DNA (see table 3).

**Table 3. Mixture of human male and female DNA.**

| **Sample No.** | **Male DNA [ng in 5 µL]** | **Female DNA [ng in 5 µL]** | **Ratio Femal DNA/male DNA** |
|---|---|---|---|
| **1** | **0.25** | **0.25** | **1** |
| **2** | **0.25** | **0.5** | **2** |
| **3** | **0. 25** | **1** | **4** |
| **4** | **0. 25** | **2** | **8** |
| **5** | **0.25** | **4** | **16** |
| **6** | **0.25** | **8** | **32** |
| **7** | **0.25** | **16** | **64** |
| **8** | **0.25** | **32** | **128** |
| **9** | **0.25** | **64** | **256** |
| **10** | **0.25** | **128** | **512** |
| **11** | **0.25** | **256** | **1024** |
| **12** | **0.25** | **512** | **2048** |
| **13** | **0.25** | **1024** | **4096** |

### EXAMPLE 2

Preparation of degraded DNA for the test kit:
Again, all work was done under the special clean room conditions outlined above. DNA preparations as discribed in Example 1 were used. Preparation of degraded DNA was achieved by deoxribonuclease (DNAse) I (Fermentas Life Sciences, St. Leon-Rot, Germany) treatment in a Mn²⁺-buffered (10 mM Tris-HCl, pH 7.5 at 25 °C; 1 mM CaCl₂, 10 mM MnCl₂) solution. Pancreatic DNAse I normally introduces single-strand nicks into double-stranded DNA. In the presence of Mn²⁺, DNAse I cleaves both strands of DNA at approximately the same site to yield fragments of DNA that are blunt-ended or that have protruding termini only one or two nucleotides in length. The reaction consists in a total volume of 230 µL of 37.5 µg genomic DNA, the buffer components mentioned above and 0.001 Units/µL enzyme. DNA and buffer solution were prepared separately (207 µL per reaction) from the enzyme dilution (freshly diluted from the storage stock of 1 Unit/µL to 0.01 Unit/µL in deionized water) and separately equilibrated at 37 °C for 5 min. The enzyme reaction was then started by the addition of 23 µL diluted enzyme to 207 µL DNA and buffer solution and incubated at 37 °C. Aliquotes of 23 µL were withdrawn at different time points (2, 3, 4, 5, 7, 10, 15 min) and stopped by mixing with 9.2 µL 50 mM EDTA-solution (wt./vol. in deionized water) followed by an incubation for 10 min at 75 °C. After cooling to room temperature 11 µL of the samples were mixed with 2 µL gel loading buffer [15 % Ficoll 400 (wt./vol;) and 0.25 mg/mL bromphenol blue in deionized water; both
chemicals were from Sigma Aldrich Chemie GmbH, Munich, Germany], subjected to agarose gel electrophoresis (1.8%), stained with ethidium bromide and fluorometically detected (Sambrook et al., 1989). The results of this kinetic pre-experiment is shown in figure 1. DNA concentration was estimated and quantified by fluorometry. The ideal fragment size is between 50 bp and 250 bp or 50 bp and 150 bp or 50 bp and 120 bp. An incubation for 7 min in this experimental setting resulted in a major fraction of DNA fragments between app. 50 bp - 250 bp. These reaction conditions were than used for two further 230 µL reactions to get a larger amount of degraded DNA which was further purified after stopping by phenol/chloroform extraction and ethanol precipitation according to Sambrook et al. (1989). Again, the fragment size was estimated by agarose gele electrophoresis and fuorometry. The quality and quantity of the degraded DNA was finally determined by UV/VIS spectrometry according to example 1.

### EXAMPLE 3

A kit according to the invention was prepared making use of a microtiter plate:
The following arrangement of the samples is dedicated to the validation of a forensic multiplex PCR kits, e. g. Mentype^{®} Nonaplex-QS PCR amplification kit (Biotype AG, Dresden, Germany), in combination with the ABI Prism^{®} 3130 Genetic Analyzer (Applied Biosystems, Foster City, CA, USA). DNA-amplificates which are labelled with one to four different fluorescence dyes are generated during the multiplex-PCR, separated by denaturing capillary gel electrophoresis and analysed by a fluorescence detector with 4 or 5 spectral channels. This DNA sequencing automate possesses an autosampler in 96 well microtiter plate format. The four capillaries are arranged in a way that the samples in wells A1-D1, A2-D2, ....., A12-D12, E1-H1, E2-H2, ....., and E12-H12 are simultaneously analyzed. Variations between the performance and sensitivity of the four capillaries exist. In addition, variations in the run-to-run precision of the capillaries and the detection unit exist. The capillary array is exchanged after 100 - 500 - (1000) runs and the spectral calibration must be repeated at regular intervals (the manufacturer recommends to exchange the capillaries after 100 runs). Thus, the validation kit of this example addresses the analytical specifications of a given multiplex PCR kit and the specifications of the ABI Prism^{®} 3130 Genetic Analyzer instrument. Furthermore, it can also be used to validate different PCR cycler with respect to the specific test system.

Again, all work was done under the special clean room conditions outlined above. DNA from humans, animals and microorganisms were prepared, characterized and quantified as described in example 1. Preparation and characterization of degraded DNA was done as described in example 2.

All human DNAs which were used in this kit were typed for autosomal and gonosomal STR-Marker which are currently used in forensics, paternity testing and chimerism analysis after bone marrow transplantation. Furthermore, two deletion/insertion polymorphisms (3 bp and 6 bp) within the amelogenin gene which are commonly used for sex genotyping were analyzed. For this purpose, commercial PCR amplification kits were used according to the instructions of the manufacturers. The kits and genetic markers are outlined in table 4.

**Table 4. Commercial multiplex PCR kits, which were used to genotype human DNAs for forensic STR-markers and sex. ABI (Applied Biosystems, Foster City, CA, USA), BT (Biotype AG, Dresden, Germany).**

| **Kit name** | **Supplier** | **Amelogein and STR-marker name** |
|---|---|---|
| **Mentype^{®} Argus X-8 PCR Amplification Kit** | **BT** | **Amelogenin (6 bp DIP), DXS8378, HPRTB (HPRT), DXS7423, DXS7132, DXS10134, DXS10074, DXS10101, DXS10135** |
| **AmpF**ℓ**STR**^{®} **Yfiler™ PCR Amplification Kit** | **ABI** | **DYS19, DYS385a/b, DYS389I/II, DYS390, DYS391, DYS392, DYS393, DYS438, DYS439, DYS437, DYS448, DYS456, DYS458, DYS635 (Y GATA C4) and Y GATA H4** |
| **Mentype^{®} Nonaplex^{QS} PCR Amplification Kit** | **BT** | **Amelogenin (3 bp DIP), D3S1358, TH01 (TC11, HUMTH01), SE33 (ACTBP2), vWA (vWF, HumVWA), FGA (FIBRA), D18S51, D8S1179, D21S11** |
| **AmpF**ℓ**fSTR^{®} Identifiler^{®} PCR Amplification Kit** | **ABI** | **Amelogenin, D3S1358, TH01 (TC11, HUMTH01), vWA (uWF, HumVWA), FGA (FIBRA), D18S51, DBS1179, D21S11, TPOX (hTPO, TPO), CSF1PO, D13S317, D16S539, D5S818, D7S820** |
| **Humantype Chimera^{®} PCR Amplification Kit** | **BT** | **Amelogenin, D2S1360, D3S1744, D4S2366, D5S2500, D6S474, D7S1517, D8S1132, D10S2325, D12S391, D18S51, D21S2055, SE33 (ACTBP2)** |
| **AmpF**ℓ**STR^{®} SGM+^{®} PCR Amplification Kit** | **ABI** | **Amelogenin, D2S1338, D3S1358, D8S1179, D16S539, D18S51, D19S433, D21S11, FGA (FIBRA), TH01 (TC11, HUMTH01), vWA (vWF, HumVWA)** |

The following schema was pipetted with a Corbett CAS-1200™ automated PCR setup robot and Tecan^{®} Genesis^{®} compatible 50 µL and/or 200 µL conductive steril filter tips (Corbett Biosciences, Mortlake, NSW, Australia). Further details are outlined in table 5, the pipetting schema is illustrated in figure 2. Appropriate stock solutions of the DNA-samples were prepared in TTE-buffer. The pipetting volume was always set to 10 µL to achieve reliable precision. A sample volume of 10 µL is also supported by all commercial STR genotyping kits which are currently available. Thus, master mixes of the kits can be directly pipetted to the validation plate. *Optical 96-well MicroAmp*^{™} reaction plates (Applied Biosystems, Foster City, CA, USA) were used as reaction flasks.
1) First group of containers: Test for species specificity
   This included one replicate of DNA from several pets and agricultural animals which live in close relation to men and/or are the source of meat. Furthermore, a mixture of microbial DNA from environmental and human flora was used.
2) Second group of containers: Analytical sensitivity
   A dilution series with defined amounts of a male DNA was performed as outlined in table 5. The arrangement of four replicates (see figure 2) allows to calculate the mean and variation between the four capillaries of the ABI Prism^{®} 3130 Genetic Analyzer.
3) Third group of containers: Blanc without DNA
   The arrangement of four replicates (see figure 2) allows to calculate the mean and variation of the baseline between the four capillaries of the ABI Prism^{®} 3130 Genetic Analyzer.
4) Fourth group of containers: Human DNA mixture study
   DNA from a male and a female volunteer was pipetted in different ratios (see table 5). Two replicates were designed. To facilitate a semi quantitative analysis with adequate precision all samples were analysed by the same capillary (see figure 2).
5) Fifth group of containers: Test of PCR inhibitors
   Theses samples were arranged in two replicates and all were analyzed by the same capillary to reduce inter-capillary variations. Stock solutions for the inhibitors hemin chloride (780 µM; Carl Roth GmbH & Co. KG, Karlsruhe, Germany), humic acid (1 mg/mL; Carl Roth GmbH & Co. KG, Karlsruhe, Germany) and bile salts (20 mM; Sigma Aldrich Chemie GmbH, Munich, Germany) were prepared in 10 mM NaOH, the stock solution for indigo carmine (100 mM; Fluka Chemie AG, Buchs, Switzerland) was prepared in deionized water. A total amount of 0.5 ng/10 µL male DNA per well, which was identical to the second group of containers (control without inhibitors), was supplemented with two different concentrations of the inhibitors, respectively. The concentrations of the inhibitors within the 10 µL solutions pipetted per well were: Hemine 400 µM (concentration 1) and 200 µM (concentration 2), humic acid 100 ng/µL (concentration 1) and 50 ng/µL (concentration 2), bile salts 2.5 mM (concentration 1) and 1.3 mM (concentration 2), and indigo carmine 20 mM (concentration 1) and 10 mM (concentration 2).
6) Sixth group of containers: Precision of allele calling
   These 12 containers include per microtiter well 0.5 ng/10 µL of genomic DNA from male and female volunteers which had been pre-genotyped for common forensic STR and amelogenin makers as outlined above.
7) Seventh group of containers: Degraded DNA
   Pre-genotyped human genomic DNA was degraded according to example 2. Two replicates of 2 ng/10 µL, 1 µg/10 µL and 0.5 ng/10 µL, respectively, were pipetted per microtiter well.
8) Eighth group of containers: Forensic stain analysis
   These were 8 empty microtiter wells were dedicated for the comparative validation of laboratory specific DNA isolation methods in comparison to the documented DNA isolation method of the inventive kit. DNA isolation methods are very critical for forensic stain analysis. The arrangement of these containers at position A12-H12 of the 96-well micro-titer plate) allows the customer using an 8-canal pipette to pipette his own samples without cross contamination to the rest of the validation plate.

Two different approaches were applied to preserve the validation kit for long time storage. The first consisted of sealing the micro-titer plates filled with 10 µL samples in TTE-buffer with *Adhesive PCR Foil Seals* (AB-0626, Thermo Fisher Scientific, Hudson, NA, USA) followed by standard freezing at -20 °C. The second approach took advantage of lyophilization (Lyovac GT2, Amsco Finn-Aqua GmbH, Huerth, Germany) prior sealing as outlined above. The lyophilized and sealed micro-titer plates were further wrapped together with a dry bag (TA Mini Bag, Tropack Packmittel GmbH, Lahnau-Waldgirmes, Germay) into an aluminium bag (BN 5695401352, Stroebel GmbH, Langenzenn, Germany) and sealed with a vacuum packaging machine *Boss NT21* (Helmut Boss Verpackungsmaschinen KG, Bad Homburg, Germany). The ladder can be stored at room temperature. In both cases, the kits were stable for at least one year.

The validation kit was accompanied by a certificate of analysis in compliance to *DIN EN ISO 9000:2005.* This necessitates that all critical analytical parameter like DNA quality, DNA quantity and genotypes of the DNA samples are clearly documented with the assay used and its limits. In some embodiments it is important to provide the genotypes of DNA standards separately after the validation analysis. This can be arranged by sending the certificates in a separate letter or by the software and/or internet solutions mentioned above. This approach is especially important for benchmarking and ring trails between different laboratories.

**Table 5: Sample list for the microtiter plate of example 3. The pipetting schema is shown in figure 2.**

| **Sample number** | **Group of containers** | **Validation category** | **DNA source and further** | **number of DNA samples per well** | **DNA amount per well** | **number of samples or replicates** |
|---|---|---|---|---|---|---|
| **1** | **First** | **Species specificity** | **Equus *cabal*/*us*** | **One species** | **2.5 ng** | **1** |
| **2** | **First** | **Species specificity** | ***Bos taurus*** | **One species** | **2.5 ng** | **1** |
| **3** | **First** | **Species specificity** | **Felis *catus*** | **One species** | **2.5 ng** | **1** |
| **4** | **First** | **Species specificity** | ***Canis lupus familiaris*** | **One species** | **2.5 ng** | **1** |
| **5** | **First** | **Species specificity** | ***Mus musculus*** | **One species** | **2.5 ng** | **1** |
| **6** | **First** | **Species specificity** | ***Rattus norvegicus*** | **One species** | **2.5 ng** | **1** |
| **7** | **First** | **Species specificity** | ***Oryctolagus cuniculus*** | **One species** | **2.5 ng** | **1** |
| **8** | **First** | **Species specificity** | ***Ovis aries*** | **One species** | **2.5 ng** | **1** |
| **9** | **First** | **Species specificity** | ***Mesocricetus auratus*** | **One species** | **2.5 ng** | **1** |
| **10** | **First** | **Species specificity** | **Sus *scrofa*** | **One species** | **2.5 ng** | **1** |
| **11** | **First** | **Species specificity** | ***Gallus gallus*** | **One species** | **2.5 ng** | **1** |
| **12** | **First** | **Species specificity** | ***Acinetobacterjohnsonii, Aspergillus niger, Bacillus subtilis, Bifidobacterium longum, Candida albicans, Corynebacterium singulare, Enterococcus faecalis, Escherichia coli, Lactobacillus crispatus, Lactobacillus rhamnosus,Proprionibacterium acnis, Pseudomonas stuzeri,* Saccharomyces *cerevisiae, Staphylococcus aureus, Staphylococcus epidermitis,* Streptococcus *mitis*** | **Microbial DNA pool of 16 different species** | **Equivalent of app. 10⁵ genome copies** | **1** |
| **13-16** | **Second** | **Analytical sensitivity** | **Human male DNA** | **One person** | **2.0 ng** | **4** |
| **17-20** | **Second** | **Analytical sensitivity** | **Human male DNA** | **One person** | **1.0 ng** | **4** |
| **21-24** | **Second** | **Analytical sensitivity** | **Human male DNA** | **One person** | **0.5 ng** | **4** |
| **25-28** | **Second** | **Analytical sensitivity** | **Human male DNA** | **One person** | **0.25 ng** | **4** |
| **29-32** | **Second** | **Analytical sensitivity** | **Human male DNA** | **One person** | **0.125 ng** | **4** |
| **33-36** | **Second** | **Analytical sensitivity** | **Human male DNA** | **One person** | **0.0625 ng** | **4** |
| **37-40** | **Third** | **Blanc** | **TTE buffer** | **Non** | **0 ng** | **4** |
| **41-42** | **Fourth** | **Human DNA mixture study** | **DNA male: DNA female = 1 : 0** | **Two persons** | **1 ng** | **2** |
| **43-44** | **Fourth** | **Human DNA mixture study** | **DNA male: DNA female = 1 : 1** | **Two persons** | **1 ng** | **2** |
| **45-46** | **Fourth** | **Human DNA mixture study** | **DNA male: DNA female = 1 : 3** | **Two persons** | **1 ng** | **2** |
| **47-48** | **Fourth** | **Human DNA mixture study** | **DNA male: DNA female = 1 : 7** | **Two persons** | **1 ng** | **2** |
| **49-50** | **Fourth** | **Human DNA mixture study** | **DNA male : DNA female = 1 : 10** | **Two persons** | **1 ng** | **2** |
| **51-52** | **Fourth** | **Human DNA mixture study** | **DNA male : DNA female = 1 : 15** | **Two persons** | **1 ng** | **2** |
| **53-54** | **Fourth** | **Human DNA mixture study** | **DNA male : DNA female = 0 : 1** | **Two persons** | **1 ng** | **2** |
| **55-56** | **Fifth** | **PCR Inhibitors** | **Human male DNA plus humic acid, concentration 1** | **One person** | **0.5 ng** | **2** |
| **57-58** | **Fifth** | **PCR Inhibitors** | **Human male DNA plus humic acid, concentration 2** | **One person** | **0.5 ng** | **2** |
| **59-60** | **Fifth** | **PCR Inhibitors** | **Human male DNA plus heme, concentration 1** | **One person** | **0.5 ng** | **2** |
| **61-62** | **Fifth** | **PCR Inhibitors** | **Human male DNA plus heme, concentration 2** | **One person** | **0.5 ng** | **2** |
| **63-64** | **Fifth** | **PCR Inhibitors** | **Human male DNA plus indigo carmine, concentration 1** | **One person** | **0.5 ng** | **2** |
| **65-66** | **Fifth** | **PCR Inhibitors** | **Human male DNA plus indigo carmine, concentration 2** | **One person** | **0.5 ng** | **2** |
| **67-68** | **Fifth** | **PCR Inhibitors** | **Human male DNA plus bile salt mixture, concentration 1** | **One person** | **0.5 ng** | **2** |
| **69-70** | **Fifth** | **PCR Inhibitors** | **Human male DNA plus bile salt mixture, concentration 2** | **One person** | **0.5 ng** | **2** |
| **71-82** | **Sixth** | **Precision of allele calling** | **Human DNA of given individuals (female and male)** | **One person** | **0.5 ng** | **12 x 1** |
| **83-84** | **Seventh** | **Degraded DNA** | **Human DNA degraded according to example 2** | **One person** | **2 ng** | **2** |
| **85-86** | **Seventh** | **Degraded DNA** | **Human DNA degraded according to example 2** | **One person** | **1 ng** | **2** |
| **87-88** | **Seventh** | **Degraded DNA** | **Human DNA degraded according to example 2** | **One person** | **0.5 ng** | **2** |
| **89-96** | **Eighth** | **Forensic stain analysis** | **Samples prepared by the customer** | **Customer defined** | **Customer defined** | **8 x 1** |

## Claims

1. Kit comprising,
a. a first group of containers, comprising two or more containers with human genomic DNA, wherein the human genomic DNA in each of the two or more containers is of different human origin and the amount of DNA in the container is defined;
b. a second group of containers, comprising two or more containers with human genomic DNA, wherein the concentration of the DNA varies between the containers and the concentration of the DNA in the container is defined;
c. a third group of containers, comprising two or more containers with human genomic DNA, wherein the human genomic DNA in each container is a mixture of genomic DNA and is from at least two different individuals.

2. Kit according to claim 1, wherein the first group of containers comprises two or more containers each comprising human genomic DNA, wherein at least one DNA is from a male individual and one DNA is from a female individual.

3. Kit according to claims 1 or 2, wherein the human genomic DNA in the second group of containers has a quantity of between 5 pg and 10 ng.

4. Kit according to claims 1 to 3, wherein the mixture of DNAs in the third group of containers is has a ratio of between 1 to 100 and 100 to 1, wherein the larger fraction has a quantity of between 50 pg and 2 ng.

5. Kit according to claims 1 to 4, wherein the mixture of DNAs in the third group of containers is has a ratio of between 1 to 50 and 50 to 1, wherein the larger fraction has a quantity of between 200 pg and 800 pg.

6. Kit according to claims 1 to 5, wherein the third group of containers comprises at least one container which carries an equal amount of said two genomic DNAs.

7. Kit according to claims 1 to 6, wherein the kit comprises a fourth group of containers and this group comprises containers with human genomic DNA and an amplification reaction inhibitor.

8. Kit according to claim 7, wherein the polymerase chain reaction inhibitor is selected from the group comprising humic acid, hemin (chloro [3,7,12,17-tetramethyl-3,13-divinylporphyrin-2,18-dipropanoato(2-)] iron(III)), indigo and bile salts (equimolar mixture of sodium salts of cholic acid and desoxycholic acid).

9. Kit according to claims 7 to 8, wherein the fourth group of containers comprises at least one container with human genomic DNA from a male individual and one container with human genomic DNA from a female individual.

10. Kit according to claims 1 to 9, wherein the kit additionally comprises a fifth group of containers comprising at least one container with a first non-human genomic DNA sample.

11. Kit according to claim 10, wherein the non-human genomic DNA sample is selected from the group of dog, cat, bovine, horse, sheep, chicken, gorilla, orang-utan, chimpanzee, macaque, *E. coli,* and *Candida albicans.*

12. Kit according to any of claims 1 to 11, wherein the kit additionally comprises a sixth group of containers comprising at least one container with a first human genomic DNA sample which is degraded.

13. Kit according to claim 12, wherein the sixth group comprises more than one container, wherein each container comprises a human genomic DNA sample and wherein each genomic DNA sample has a different state of degradation.

14. Kit according any of claims 1 to 13, wherein the kit additionally comprises a seventh group of containers comprising at least one container with a first human genomic DNA sample as well as a second human genomic DNA sample, wherein the second human genomic DNA is from a different individual than the first sample and the second sample is degraded.

15. Kit according any of claims 1 to 14, wherein the kit additionally comprises an eighth group of containers comprising at least one container with no DNA but either water or a buffer or another solution which may be added to an amplification reaction.

16. Kit according to any of the following claims wherein the containers are wells in a micro titerplate and the plate has 6, 24, 96, 384 or even 1536 sample wells arranged in a 2:3 rectangular matrix.
